Europäisches Patentamt

European Patent Office

Office européen des brevets

(11) Veröffentlichungsnummer : **0 348 832 B1**

(12) # EUROPÄISCHE PATENTSCHRIFT

(45) Veröffentlichungstag der Patentschrift :
26.08.92 Patentblatt 92/35

(51) Int. Cl.$^5$ : **C07C 209/26, C07C 211/19**

(21) Anmeldenummer : **89111443.1**

(22) Anmeldetag : **23.06.89**

(54) **Verfahren zur Herstellung von 3(4),8(9)-Bis(aminomethyl)-tricyclo[5.2.1.0,26]-decan.**

(30) Priorität : **30.06.88 DE 3822038**

(43) Veröffentlichungstag der Anmeldung :
**03.01.90 Patentblatt 90/01**

(45) Bekanntmachung des Hinweises auf die
Patenterteilung :
**26.08.92 Patentblatt 92/35**

(84) Benannte Vertragsstaaten :
**AT DE ES FR GB SE**

(56) Entgegenhaltungen :
**EP-A- 0 026 983**
**GB-A- 1 170 226**

(73) Patentinhaber : **HOECHST
AKTIENGESELLSCHAFT
Postfach 80 03 20
W-6230 Frankfurt am Main 80 (DE)**

(72) Erfinder : **Lukas, Rainer, Dr. Dipl.-Chem.
Holunderweg 13
W-4300 Essen 1 (DE)**
Erfinder : **Mathieu, Klaus
Dinslakener Strasse 123
W-4200 Oberhausen 11 (DE)**
Erfinder : **Thönnessen, Franz, Dr. Dipl.-Chem.
Lützowstrasse 49
W-4200 Oberhausen 11 (DE)**
Erfinder : **Dämbkes, Georg, Dr. Dipl.-Chem.
Nibelungenstrasse 65
W-4220 Dinslaken (DE)**
Erfinder : **Bach, Hanswilhelm, Dr. Dipl.-Chem.
Alleestrasse 56
W-4100 Duisburg 11 (DE)**

**Beschreibung**

Die Erfindung betrifft ein Verfahren zur Herstellung von 3(4) ,8(9)-Bis(aminomethyl)-tricyclo[5.2.1.0$^{2,6}$]-decan, eine Verbindung, die im folgenden auch kurz TCD-diamin genannt wird.

TCD-diamin findet als wertvolles Zwischenprodukt in zahlreichen technisch ausgeübten Synthesen Anwendung. So wird es z.B. bei der Gewinnung von Polyurethanen oder als Härter für Epoxidharze eingesetzt.

Zur Herstellung des TCD-diamins geht man üblicherweise von Dicyclopentadien aus. Man erhält diesen Ausgangsstoff durch Dimerisierung von Cyclopentadien, die bereits bei Raumtemperatur erfolgt und durch Katalysatoren beschleunigt werden kann. Das dimere Cyclopentadien wird durch Hydroformylierung, d.h. durch Umsetzung mit Kohlenmonoxid und Wasserstoff in Gegenwart von Katalysatoren, z. B. Kobalt oder Rhodium, die als feinverteilte Metalle oder als Verbindungen eingesetzt werden, in den entsprechenden Tricyclodecandialdehyd (TCD-dial) übergeführt. Ein geeignetes Verfahren ist z. B. in der britischen Patentschrift 11 70 226 beschrieben.

Die Umwandlung des TCD-dials in das TCD-diamin erfolgt in bekannter Weise durch reduktive Aminierung der Formylgruppen. Hierzu wird der Dialdehyd in Gegenwart eines Hydrierkatalysators gegebenenfalls nach vorheriger Umsetzung mit einem primären Amin mit Ammoniak und Wasserstoff behandelt. Geeignete Reaktionsbedingungen sind Temperaturen von 80 bis 150°C und Drücke von 5 bis 12 MPa. Hydrierkatalysatoren auf Nickelbasis haben sich für diesen Zweck bewährt.

Es ist ein Nachteil des bekannten Verfahrens, daß die Abtrennung des Dialdehyds aus dem Reaktionsprodukt der Hydroformylierung erhebliche Schwierigkeiten bereitet. Die Ursache hierfür ist die hohe Reaktivität der Dialdehyde. Sie reagieren nicht nur miteinander unter Bildung höhermolekularer Folgeprodukte, sondern auch mit weiteren im Reaktionsprodukt enthaltenen Verbindungen und gehen damit der angestrebten Umsetzung verloren. Man hat versucht diesen Mangel unter anderem durch besonders schonende Destillation z. B. Dünnschichtdestillation unter vermindertem Druck zu beheben. Aber auch die Einhaltung solcher Vorsichtsmaßnahmen schließt die unerwünschten Nebenreaktionen nicht aus, abgesehen davon, daß die geeigneten Destillationsverfahren aufwendig sind und die Wirtschaftlichkeit der Synthese infrage stellen.

Es bestand daher die Aufgabe ein Verfahren zu entwickeln, das es erlaubt TCD-diamin unter Vermeidung der geschilderten Probleme in hoher Ausbeute aus Dicyclopentadien herzustellen.

Erfindungsgemäß wird diese Aufgabe gelöst durch ein Verfahren zur Herstellung von 3(4), 8(9)-Bis(aminomethyl)tricyclo[5.2.1.0$^{2,6}$]-decan durch Hydroformylierung von Dicyclopentadien in Gegenwart von Rhodiumkatalysatoren und anschließende Reaktion des erhaltenen Tricyclodecandialdehyds mit Wasserstoff und Ammoniak in Gegenwart eines Hydrierkatalysators (reduktive Aminierung). Es ist dadurch gekennzeichnet, daß das Hydroformylierungsprodukt ohne vorherige Aufarbeitung, insbesondere ohne Abtrennung des Rhodiumkatalysators, vorzugsweise nach Umsetzung mit einem primären Amin, reduktiv aminiert wird.

Überraschenderweise hat sich gezeigt, daß es entsprechend dem neuen Verfahren nicht erforderlich ist, vor der reduktiven Aminierung den Tricyclodecandialdehyd zu isolieren und den Hydroformylierungskatalysator aus dem Reaktionsgemisch zu entfernen. Hierbei ist zu berücksichtigen, daß der Hydroformylierungskatalysator die Weiterreaktion des zunächst gebildeten TCD-dials zu höhermolekularen Verbindungen begünstigt.

Ausgangsstoff für die Herstellung von TCD-diamin nach dem neuen Verfahren ist Dicyclopentadien. Es kann in handelsüblicher Form, also ohne vorheriger Reinigung eingesetzt werden.

Die Hydroformylierung des Dicyclopentadiens mit Kohlenmonoxid und Wasserstoff erfolgt in an sich bekannter Weise in Gegenwart oder auch in Abwesenheit eines Lösungsmittels bei Temperaturen von 100 bis 200°C und bei Drücken von 20 bis 30 MPa. Die Reaktion wird in Gegenwart von Rhodium als Katalysator durchgeführt. Man setzt es als Metall in feinverteilter Form ein. Bevorzugt verwendet man jedoch Verbindungen wie Dirhodiumtrioxid oder Rhodiumsalze schwacher organischer Säuren, z. B. der Essigsäure oder der 2-Ethylhexansäure. Die Konzentration des Rhodiums kann zwischen 20 und 100 mg Rh/kg Dicyclopentadien variieren, der bevorzugte Bereich liegt zwischen 20 und 50 mg Rh/kg Diolefin. Die Hydroformylierung wird absatzweise oder bevorzugt kontinuierlich durchgeführt.

Eine Aufarbeitung des Reaktionsgemisches insbesondere die Abtrennung des Dialdehyds und/oder die Entfernung des Katalysators ist nicht erforderlich. Das Hydroformylierungsgemisch kann vielmehr so, wie es erhalten wurde, gegebenenfalls also auch zusammen mit einem Lösungsmittel im zweiten Reaktionsschritt reduktiv aminiert werden.

Unter reduktiver Aminierung versteht man die Reaktion des Dialdehyds mit Wasserstoff und Ammoniak in Gegenwart eines Hydrierkatalysators. Nach einer bevorzugten Ausführungsform der Erfindung setzt man den Dialdehyd zunächst mit einem primären Amin unter Bildung eines Diazomethins um und behandelt darauf das Diazomethin mit Wasserstoff und Ammoniak in Gegenwart eines Hydrierkatalyators. Auch diese Reaktion wird im Sinne der Erfindung als reduktive Aminierung bezeichnet.

2

Zur Bildung des Diazomethins gibt man zu dem nicht aufgearbeiteten Hydroformylierungsgemisch je mol Tricyclodecandialdehyd 2 bis 6 mol, insbesondere 3 bis 4 mol eines primären Amins. Die Reaktion zwischen den Ausgangsstoffen läuft bereits bei Raumtemperatur ab, sie kann durch Erhitzen auf 20 bis 60, insbesondere 30 bis 50°C beschleunigt werden. Als primäre Amine geeignet sind Amine mit 2 bis 10 Kohlenstoffatomen im Molekül. Mit besonderem Erfolg werden Amine mit 3 bis 5 Kohlenstoffatomen im Molekül, vorzugsweise n-Butylamin, eingesetzt.

Die reduktive Aminierung des Dialdehyds oder des Diazomethins wird zweckmäßig bei Temperaturen von 80 bis 150°C, vorzugsweise 120 bis 140°C durchgeführt. Der Wasserstoffdruck im Reaktionsgefäß beträgt bei der Reaktionstemperatur 5 bis 12 und insbesondere 8 bis 10 MPa.

Als Hydrierkatalysatoren werden Nickel oder Kobalt verwendet, entweder in Form von Raney-Nickel bzw. Raney-Kobalt oder auch in Form entsprechender Trägerkatalysatoren. Ein bevorzugter Katalysator enthält 50 bis 60 Gew.-% Nickel auf Kieselgur.

Ammoniak soll zweckmäßigerweise im Überschuß angewendet werden. Je mol Formylgruppe bzw. je mol Diazomethingruppe sind mindestens 2 mol Ammoniak erforderlich, vorzugsweise werden 4 bis 5 mol Ammoniak eingesetzt.

Die reduktive Aminierung des Dialdehyds oder des Diazomethins kann in Abwesenheit von Lösungsmitteln durchgeführt werden, üblicherweise wirkt das Endprodukt selbst als Lösungsmittel. Bei kleinen diskontinuierlichen Ansätzen ist es jedoch vorteilhaft, in einem Lösungsmittel zu arbeiten. Besonders gute Ergebnisse werden bei Verwendung von Tetrahydrofuran, Isobutanol, Butanol oder Isopropanol als Lösungsmittel erzielt.

Das Gemisch der isomeren Diamine wird in guten Ausbeuten erhalten. Nur ein kleiner Teil der Dialdehyde geht in höhermolekulare Kondensationsprodukte über. Sie sind im Reaktionsgemisch gelöst und stören die Produktentnahme aus dem Reaktor und eine störungsfreie Aufarbeitung der Rohprodukte nicht. Im Destillationsrückstand bleibt der Hydrierkatalysator zurück, auf den sich das in der Hydroformylierungsstufe eingesetzte Rhodium nahezu vollständig abscheidet. Es kann nach bekannten Verfahren zurückgewonnen werden.

Zur Abtrennung der isomeren TCD-diamine destilliert man das Reaktionsgemisch, vorzugsweise unter vermindertem Druck. Man erhält die Verbindungen als farblose Flüssigkeit, die bei etwa 310°C siedet.

In den folgenden Beispielen wird das neue Verfahren näher erläutert. Selbstverständlich ist nicht beabsichtigt, die Erfindung auf diese speziellen Ausführungsformen zu beschränken.

Beispiel 1

(a) Hydroformylierung

In einem Rührautoklav erhitzt man eine Lösung von 70 Gew.-Teilen Dicyclopentadien in 30 Gew.-Teilen Toluol auf 135°C. Darauf wird in Gegenwart von 50 Gew.-ppm Rhodium (eingesetzt als Rh-2-ethylhexanoat) während 3 Stunden Synthesegas ($H_2 : CO = 1 : 1$) unter einem Druck von 25 MPa in die Lösung geleitet. Das Reaktionsprodukt besteht zu 42 Gew.-% aus Tricyclodecandialdehyd, zu 44 Gew.-% aus Toluol, der Rest Tricyclodecanmonoaldehyd (11 Gew.-%) sowie leichtsiedende und hochsiedende Komponenten.

(b) Aminierende Hydrierung

In einem Autoklav dosiert man zu 30 Gew.-Teilen n-Butylamin über einen Zeitraum von 1 Stunde und unter Einhaltung einer Temperatur von maximal 60°C 30 Gew.-Teile des TDC-dial enthaltenden Hydroformylierungsrohproduktes nach (a) und rührt anschließend weitere 30 Minuten bei 40°C. Das das Diazomethin des TDC-dials enthaltende Reaktionsprodukt mit einem Rhodiumgehalt von 18,5 Gew.-ppm wird zusammen mit Ammoniak und Wasserstoff kontinuierlich an einem als Festbett angeordneten Ni-Katalyator hydriert. Die Umsetzung erfolgt bei einer Reaktionstemperatur von 130°C, einem Wasserstoffdruck von 8 MPa, einem $NH_3$/Diazomethin-Molverhältnis von 50 : 1 und einer Raumgeschwindigkeit von 0,4 Volumen Produktgemisch je Volumen Katalysator und Stunde. Nach der $NH_3$-Abtrennung enthält das Reaktionsprodukt 27 Gew.-% TCD-diamin, 44 Gew.-% n-Butylamin, 16 Gew.-% Toluol, der Rest sind Isomere und Nachläufe. Anschließende erfolgt die destillative Aufarbeitung zum Reinprodukt. Der Rhodiumaustrag nach dem Hydrierreaktor beträgt 0,01 Gew.ppm, d.h. 99,95 % des eingesetzten Rhodiums werden auf dem Katalysator zurückgehalten.

Beispiel 2

Die Hydroformylierung des Dicyclopentadiens wird wie in Beispiel 1 durchgeführt. Nach Abtrennung des Vorlaufs über einen Dünnschichtverdampfer enthält das Hydroformylierungsprodukt 74 Gew.-% TCD-dial und 37 mg Rh/kg Produkt.

30 Gew.-Teile Hydroformylierungsprodukt werden mit 30 Gew.-Teilen n-Butylamin bei 40°C und einem Durchsatz von 0,2 Volumen Produkt je Reaktor-Volumen und Stunde in einem Reaktionsrohr kontinuierlich zum Diazomethin umgesetzt. Anschließend setzt man bei 130°C, unter einem Druck von 8 MPa Wasserstoff, bei einem $NH_3$/Diazomethin-Molverhältnis von 10 : 1 und einer Raumgeschwindigkeit von 0,3 Volumen Produktgemisch je Volumen Katalysator und Stunde kontinuierlich an einem Ni-Katalysator zum TCD-diamin um. Der Rhodiumgehalt im Reaktionsprodukt beträgt weniger als 0,1 Gew.-ppm. Zur Reinigung wird das TCD-diamin destilliert.

**Patentansprüche**

1. Verfahren zur Herstellung von 3(4),8(9)-Bis(aminomethyl)-tricyclo[5.2.1.0$^{2,6}$]-decan durch Hydroformylierung von Dicyclopentadien in Gegenwart von Rhodiumkatalysatoren und anschließende Reaktion des erhaltenen Tricyclodecandialdehyds mit Wasserstoff und Ammoniak in Gegenwart eines Hydrierkatalysators (reduktive Aminierung), dadurch gekennzeichnet, daß das Hydroformylierungsprodukt ohne vorherige Aufarbeitung, insbesondere ohne Abtrennung des Rhodiumkatalysators, vorzugsweise nach Umsetzung mit einem primären Amin reduktiv aminiert wird.

2. Verfahren nach Anspruch 1, dadurch gekennzeichnet, daß die Hydroformylierung des Dicyclopentadiens bei Temperaturen von 100 bis 200°C und Drücken von 20 bis 30 MPa in Gegenwart oder in Abwesenheit eines Lösungsmittels erfolgt.

3. Verfahren nach Anspruch 1 oder 2, dadurch gekennzeichnet, daß das Rhodium in einer Konzentration von 20 bis 100 mg/kg Dicyclopentadien, vorzugsweise 20 bis 50 mg/kg Dicyclopentadien eingesetzt wird.

4. Verfahren nach einem oder mehreren der Ansprüche 1 bis 3, dadurch gekennzeichnet, daß man je mol im Hydroformylierungsprodukt enthaltenem Tricyclodecan-dialdehyd 2 bis 6 mol, insbesondere 3 bis 4 mol eines primären Amins einsetzt.

5. Verfahren nach einem oder mehreren der Ansprüche 1 bis 4, dadurch gekennzeichnet, daß die primären Amine 2 bis 10, insbesondere 3 bis 6 Kohlenstoffatome im Molekül enthalten.

6. Verfahren nach einem oder mehreren der Ansprüche 1 bis 5, dadurch gekennzeichnet, daß die Umsetzung des Tricyclodecandialdehyds mit dem Amin bei 20 bis 60, insbesondere 30 bis 50°C erfolgt.

7. Verfahren nach einem oder mehreren der Ansprüche 1 bis 6, dadurch gekennzeichnet, daß die reduktive Aminierung des Tricyclodecandialdehyds bei 80 bis 150°C, vorzugsweise 120 bis 140°C und Drücken von 5 bis 12, insbesondere 8 bis 10 MPa durchgeführt wird.

8. Verfahren nach einem oder mehreren der Ansprüche 1 bis 7, dadurch gekennzeichnet, daß bei der reduktiven Aminierung je mol Formyl- bzw. Diazomethingruppe mindestens 2 mol, vorzugsweise 4 bis 5 mol Ammoniak eingesetzt werden.

**Claims**

1. A process for preparing 3(4),8(9)-bis(aminomethyl)tricyclo-(5.2. 1.0$^{2,6}$)decane by the hydroformylation of dicyclopentadiene in the presence of rhodium catalysts and subsequent reaction of the tricyclodecane dialdehyde obtained with hydrogen and ammonia in the presence of a hydrogenation catalyst (reductive amination), characterised in that the hydroformylation product is reductively aminated without previous work-up, in particular without separation of the rhodium catalyst, preferably after reaction with a primary amine.

2. A process according to claim 1, characterised in that the hydroformylation of the dicyclopentadiene takes place at temperatures of 100 to 200°C and pressures of 20 to 30 MPa in the presence or in the absence of a solvent.

3. A process according to claim 1 or 2, characterised in that the rhodium is used in a concentration of 20 to 100 mg/kg of dicyclopentadiene, preferably 20 to 50 mg/kg of dicyclopentadiene.

4. A process according to one or more of the claims 1 to 3, characterised in that 2 to 6 moles, in particular 3 to 4 moles of a primary amine are used per mole of tricyclodecane dialdehyde contained in the hydroformylation product.

5. A process according to one or more of the claims 1 to 4, characterised in that the primary amines contain 2 to 10, in particular 3 to 6 carbon atoms in the molecule.

6. A process according to one or more of the claims 1 to 5, characterised in that the reaction of the tricyclodecane dialdehyde with the amine takes place at 20 to 60, in particular 30 to 50°C.

7. A process according to one or more of the claims 1 to 6, characterised in that the reductive amination of the tricyclodecane dialdehyde is performed at temperatures of 80 to 150° C, preferably 120 to 140° C and pressures of 5 to 12, in particular 8 to 10 MPa.

8. A process according to one or more of the claims 1 to 7, characterised in that in the reductive amination at least 2 moles, preferably 4 to 5 moles of ammonia are used per mole of the formyl group or diazomethine group.

## Revendications

1. Procédé pour la fabrication de 3(4),8(9)-bis(aminométhyl)tricyclo[5.2.1.0$^{2,6}$]-décane par hydroformylation du dicyclopentadiène en présence de catalyseurs au rhodium et réaction ultérieure du tricyclodécanedialdéhyde obtenu avec l'hydrogène et l'ammoniac en présence d'un catalyseur d'hydrogénation (amination réductrice), caractérisé en ce que le produit d'hydroformylation est soumis à l'amination réductrice sans traitement préalable, en particulier sans séparation du catalyseur au rhodium, de préférence après réaction avec une amine primaire.

2. Procédé selon la revendication 1, caractérisé en ce que l'hydroformylation du dicyclopentadiène s'effectue à des températures de 100 à 200°C et sous des pressions de 20 à 30 MPa en présence ou en l'absence de solvant.

3. Procédé selon la revendication 1 ou 2, caractérisé en ce que le rhodium est utilisé à une concentration de 20 à 100 mg/kg de dicyclopentadiène.

4. Procédé selon une ou plusieurs des revendications 1 à 3, caractérisé en ce que l'on utilise 2 à 6 mol d'une amine primaire, en particulier 3 à 4 mol, par mole du tricyclodécanedialdéhyde contenu dans le produit d'hydroformylation.

5. Procédé selon une ou plusieurs des revendications 1 à 4, caractérisé en ce que les amines primaires contiennent 2 à 10 atomes de carbone par molécule, en particulier 3 à 6 atomes de carbone.

6. Procédé selon une ou plusieurs des revendications 1 à 5, caractérisé en ce que la réaction du tricyclodécanedialdéhyde avec l'amine s'effectue à 20-60°C, en particulier 30-50°C.

7. Procédé selon une ou plusieurs des revendications 1 à 6, caractérisé en ce que l'amination réductrice du tricyclodécanedialdéhyde est mise en oeuvre à 80-150°C, de préférence 120-140°C, et sous des pressions de 5 à 12 MPa, en particulier de 8 à 10 MPa.

8. Procédé selon une ou plusieurs des revendications 1 à 7, caractérisé en ce que l'on utilise dans l'amination réductrice au moins 2 mol d'ammoniac, de préférence 4 à 5 mol d'ammoniac, par mole de groupe formyle ou de groupe diazométhine.